(19) Europäisches Patentamt / European Patent Office / Office européen des brevets

(11) **EP 3 437 726 A1**

(12) **EUROPEAN PATENT APPLICATION**
published in accordance with Art. 153(4) EPC

(43) Date of publication:
**06.02.2019 Bulletin 2019/06**

(51) Int Cl.:
**B01D 71/02** (2006.01)    **B01D 53/22** (2006.01)
**C07C 7/144** (2006.01)    **C07C 9/18** (2006.01)

(21) Application number: **17773743.4**

(22) Date of filing: **13.02.2017**

(86) International application number:
**PCT/JP2017/005179**

(87) International publication number:
**WO 2017/169195 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.03.2016 JP 2016073134**

(71) Applicant: **Zeon Corporation**
**Tokyo 100-8246 (JP)**

(72) Inventors:
• **OMORI Shiori**
**Tokyo 100-8246 (JP)**
• **SUZUKI Takahiro**
**Tokyo 100-8246 (JP)**
• **SASANUMA Takashi**
**Tokyo 100-8246 (JP)**

(74) Representative: **Maiwald Patent- und Rechtsanwaltsgesellschaft mbH**
**Elisenhof**
**Elisenstraße 3**
**80335 München (DE)**

(54) **MEMBRANE SEPARATION METHOD AND MEMBRANE SEPARATION DEVICE**

(57)    Provided are a membrane separation method and a membrane separation device that enable membrane separation of a hydrocarbon mixture with high separation efficiency. The membrane separation method includes a step (A) of exposing a zeolite membrane to an atmosphere having a dew point of -20°C or lower and a step (B) of using the zeolite membrane to perform membrane separation of a hydrocarbon mixture after step (A). The membrane separation device includes: a membrane separation module including a housing and a zeolite membrane that is housed in the housing and is configured to perform membrane separation of a hydrocarbon mixture; a feedstock supply mechanism configured to supply the hydrocarbon mixture into the membrane separation module; and a gas supply mechanism configured to supply a gas having a dew point of -20°C or lower into a space in which the zeolite membrane is housed in the membrane separation module.

**Description**

TECHNICAL FIELD

The present disclosure relates to a membrane separation method and a membrane separation device and, in particular, relates to a membrane separation method and a membrane separation device that can suitably be used to separate one or more hydrocarbons from a hydrocarbon mixture.

BACKGROUND

Membrane separation is conventionally used as a method for low-energy separation of a specific component from a mixture of multiple components. The separation membrane that is used may, for example, be a zeolite membrane that is obtained by forming a zeolite on a support in the form of a membrane.

The performance of a zeolite membrane used in membrane separation is normally expressed by the permeation flux F and separation factor $\alpha$ of a permeate. There is demand for increasing the permeation flux F and separation factor $\alpha$ for zeolite membranes used in membrane separation.

Consequently, PTL 1, for example, proposes a technique for enabling membrane separation of a mixed gas with a high permeation flux while inhibiting reduction of the separation factor by using a zeolite membrane that has been exposed to water in membrane separation of the mixed gas after subjecting the zeolite membrane to heat treatment in an air atmosphere furnace under specific temperature conditions.

Moreover, membrane separation using zeolite membranes has been attracting interest in recent years as a method for low-energy separation of a branched hydrocarbon from a hydrocarbon mixture containing linear and branched hydrocarbons of equivalent carbon number. In membrane separation of hydrocarbon mixtures using zeolite membranes, there is demand for increasing both the permeation flux F and separation factor $\alpha$, and improving separation efficiency.

CITATION LIST

Patent Literature

PTL 1: WO 2014/069630 A1
PTL 2: JP 2015-160186 A

SUMMARY

(Technical Problem)

However, it has not been possible to sufficiently improve separation efficiency in membrane separation of a hydrocarbon mixture by a conventional membrane separation method using a zeolite membrane such as described above.

Accordingly, an objective of the present disclosure is to provide a membrane separation method and a membrane separation device that enable membrane separation of a hydrocarbon mixture with high separation efficiency.

(Solution to Problem)

The inventors conducted diligent investigation to achieve the objective set forth above. Through this investigation, the inventors discovered that by performing membrane separation of a hydrocarbon mixture using a zeolite membrane that has been exposed to an atmosphere having a dew point of -20°C or lower, the permeation flux F and separation factor $\alpha$ can both be increased, and separation efficiency can be improved, and in this manner completed the present disclosure.

Specifically, the present disclosure aims to advantageously solve the problems set forth above by disclosing a membrane separation method comprising: a step (A) of exposing a zeolite membrane to an atmosphere having a dew point of -20°C or lower; and a step (B) of using the zeolite membrane to perform membrane separation of a hydrocarbon mixture after the step (A). When a zeolite membrane that has been exposed to an atmosphere having a dew point of -20°C or lower is used as set forth above, a hydrocarbon mixture can be membrane separated with high separation efficiency.

The term "dew point" as used in the present disclosure refers to the dew point under atmospheric pressure as determined from moisture content measured by Fourier-transform infrared spectroscopy (FT-IR).

In the presently disclosed membrane separation method, temperature of the atmosphere is preferably raised in the step (A). Separation efficiency of the hydrocarbon mixture can be further improved by raising the temperature of the atmosphere to which the zeolite membrane is exposed.

In the presently disclosed membrane separation method, the atmosphere preferably has a maximum temperature of at least 100°C and not higher than 580°C in the step (A). Separation efficiency of the hydrocarbon mixture can be further improved while inhibiting loss of the zeolite membrane caused by heat when the maximum temperature of the atmosphere to which the zeolite membrane is exposed is at least 100°C and not higher than 580°C.

In the presently disclosed membrane separation method, the atmosphere is preferably an inert gas atmosphere. When the atmosphere to which the zeolite membrane is exposed is an inert gas atmosphere, reaction between the zeolite membrane and the atmosphere to which the zeolite membrane is exposed can be inhibited, and separation efficiency of the hydrocarbon mixture can be further improved.

In the presently disclosed membrane separation method, the zeolite membrane is preferably exposed to the atmosphere for 5 hours or more in the step (A). Separation efficiency of the hydrocarbon mixture can be sufficiently improved when the zeolite membrane is exposed to the atmosphere having a dew point of -20°C or lower for 5 hours or more.

Moreover, the present disclosure aims to advantageously solve the problems set forth above by disclosing a membrane separation device comprising: a membrane separation module including a housing and a zeolite membrane that is housed in the housing and is configured to perform membrane separation of a hydrocarbon mixture; a feedstock supply mechanism configured to supply the hydrocarbon mixture into the membrane separation module; and a gas supply mechanism configured to supply a gas having a dew point of -20°C or lower into a space in which the zeolite membrane is housed in the membrane separation module. Through inclusion of a gas supply mechanism as set forth above, membrane separation of the hydrocarbon mixture can be performed after bringing the gas having a dew point of -20°C or lower into contact with the zeolite membrane. Consequently, the hydrocarbon mixture can be membrane separated with high separation efficiency.

The presently disclosed membrane separation device preferably further comprises a heating device configured to heat the gas. Through inclusion of the heating device, the temperature of the gas having a dew point of -20°C or lower that is brought into contact with the zeolite membrane can be raised. Therefore, when the gas having a dew point of -20°C or lower is brought into contact with the zeolite membrane and then membrane separation of the hydrocarbon mixture is performed, the temperature of the gas having a dew point of -20°C or lower can be raised and separation efficiency of the hydrocarbon mixture can be further improved.

In the presently disclosed membrane separation device, the gas supply mechanism preferably includes the heating device. In a case in which the gas supply mechanism includes the heating device, preheated gas can be supplied into the membrane separation module. Consequently, when the heated gas is brought into contact with the zeolite membrane and then membrane separation of the hydrocarbon mixture is performed, the zeolite membrane can be uniformly heated and separation efficiency of the hydrocarbon mixture can be further improved compared to a situation in which the gas is heated inside the membrane separation module or the like.

In the presently disclosed membrane separation device, the gas is preferably an inert gas. When the gas having a dew point of -20°C or lower is brought into contact with the zeolite membrane and then membrane separation of the hydrocarbon mixture is performed, the use of an inert gas can inhibit reaction of the zeolite membrane and the gas, and can further improve separation efficiency of the hydrocarbon mixture.

(Advantageous Effect)

According to the present disclosure, it is possible to provide a membrane separation method and a membrane separation device that enable membrane separation of a hydrocarbon mixture with high separation efficiency.

BRIEF DESCRIPTION OF THE DRAWING

In the accompanying drawing,

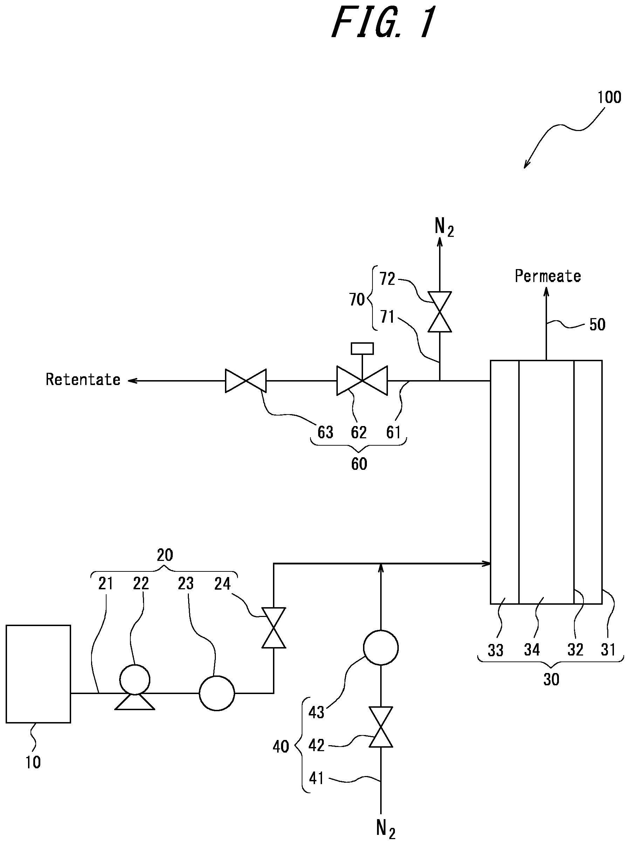

FIG. 1 illustrates an example of schematic configuration of a membrane separation device.

DETAILED DESCRIPTION

The following provides a detailed description of embodiments of the present disclosure.

A presently disclosed membrane separation method can be used in membrane separation of a hydrocarbon mixture. Moreover, a presently disclosed membrane separation device can suitably be used in membrane separation of a hydrocarbon mixture by the presently disclosed membrane separation method.

(Membrane separation method)

The presently disclosed membrane separation method is a method of membrane separating a hydrocarbon mixture using a zeolite membrane and includes a step (A) of exposing a zeolite membrane to an atmosphere having a dew point of -20°C or lower and a step (B) of using the zeolite membrane to perform membrane separation of a hydrocarbon mixture after step (A). Through use of a zeolite membrane that has been exposed to an atmosphere having a dew point of -20°C or lower in the presently disclosed membrane separation method, membrane separation of a hydrocarbon mixture can be performed with a high permeation flux and a high separation factor. Consequently, hydrocarbon compounds contained in the hydrocarbon mixture can be separated with high separation efficiency.

Although it is not clear why the permeation flux and separation factor are both improved by using a zeolite membrane that has been exposed to an atmosphere having a dew point of -20°C or lower, the reason is presumed to be as follows. Specifically, molecules of hydrophilic compounds such as water are normally adsorbed by a zeolite membrane. In an atmosphere having a high dew point such as in an air atmosphere, it is difficult to cause sufficient desorption of hydrophilic compound molecules that are adsorbed by the zeolite membrane because re-adsorption of water and the like occurs. Since hydrocarbon compounds contained in a hydrocarbon mixture are hydrophobic substances, the use of a zeolite membrane having molecules of a hydrophilic compound adsorbed thereto results in reduction of not only the permeation flux F, but also the separation factor $\alpha$. However, as a result of a zeolite membrane being exposed to an atmosphere having a dew point of -20°C or lower in the presently disclosed membrane separation method, sufficient desorption of hydrophilic compound molecules adsorbed by the zeolite membrane can be achieved, and the permeation flux F and separation factor $\alpha$ can both be increased in membrane separation of a hydrocarbon mixture.

<Hydrocarbon mixture>

The hydrocarbon mixture that is membrane separated by the presently disclosed membrane separation method may, without any specific limitations, be any mixture of a plurality of hydrocarbon compounds for which one or more hydrocarbon compounds can be separated using a zeolite membrane. Specifically, the hydrocarbon mixture may, for example, be a mixture that contains a linear hydrocarbon and a branched hydrocarbon and/or cyclic hydrocarbon of equivalent carbon number to the linear hydrocarbon. Of such mixtures, the hydrocarbon mixture is preferably a mixture that contains, as main components, a linear hydrocarbon having a carbon number of 4 and a branched hydrocarbon having a carbon number of 4 and/or a cyclic hydrocarbon having a carbon number of 4, or a mixture that contains, as main components, a linear hydrocarbon having a carbon number of 5 and a branched hydrocarbon having a carbon number of 5 and/or a cyclic hydrocarbon having a carbon number of 5, and is more preferably a mixture that contains, as main components, a linear hydrocarbon having a carbon number of 5 and a branched hydrocarbon having a carbon number of 5 and/or a cyclic hydrocarbon having a carbon number of 5.

The phrase "containing, as main components, a linear hydrocarbon and a branched hydrocarbon and/or cyclic hydrocarbon" as used in the present disclosure means that the hydrocarbon mixture comprises 50 mol% or more, in total, of the linear hydrocarbon and the branched hydrocarbon and/or cyclic hydrocarbon.

The mixture containing, as main components, a linear hydrocarbon having a carbon number of 4 and a branched hydrocarbon and/or cyclic hydrocarbon having a carbon number of 4 (hereinafter, also referred to as a "C4 hydrocarbon mixture") may, for example, be a mixture containing a linear hydrocarbon having a carbon number of 4 such as n-butane, 1-butene, 2-butene, or butadiene, and a branched hydrocarbon having a carbon number of 4 such as isobutane or isobutene and/or a cyclic hydrocarbon having a carbon number of 4 such as cyclobutane or cyclobutene. Specifically, the C4 hydrocarbon mixture may, for example, be a C4 fraction obtained as a by-product in thermal cracking of naphtha to produce ethylene or a fraction that remains after collecting at least some butadiene from this C4 fraction.

The mixture containing, as main components, a linear hydrocarbon having a carbon number of 5 and a branched hydrocarbon and/or cyclic hydrocarbon having a carbon number of 5 (hereinafter, also referred to as a "C5 hydrocarbon mixture") may, for example, be a mixture containing a linear hydrocarbon having a carbon number of 5 such as n-pentane, 1-pentene, 2-pentene, or 1,3-pentadiene, and a branched hydrocarbon having a carbon number of 5 such as isopentane, 2-methyl-1-butene, 2-methyl-2-butene, 3-methyl-1-butene, or isoprene and/or a cyclic hydrocarbon having a carbon number of 5 such as cyclopentane or cyclopentene. Specifically, the C5 hydrocarbon mixture may, for example, be a C5 fraction obtained as a by-product in thermal cracking of naphtha to produce ethylene or a fraction that remains after collecting at least some isoprene from this C5 fraction.

<Zeolite membrane>

The zeolite membrane used in the presently disclosed membrane separation method may be any zeolite membrane that can separate a desired hydrocarbon compound from a hydrocarbon mixture. Specifically, the zeolite membrane may be, but is not specifically limited to, a separation membrane that includes a porous support and a porous separation layer that is disposed on the porous support and that contains a zeolite (aluminosilicate and/or silicalite) that can separate a desired hydrocarbon compound. More specifically, in membrane separation of a C4 hydrocarbon mixture or a C5 hydrocarbon mixture, the zeolite membrane is preferably a separation membrane including a porous support and a porous separation layer that is disposed on the porous support and that contains an MFI-type zeolite (aluminosilicate and/or silicalite having an MFI structure), and is more preferably a separation membrane including a porous support and a porous separation layer that is substantially composed of an MFI-type zeolite.

The porous support may be a porous body of any material so long as it is capable of supporting a porous separation layer. Of such porous bodies, a porous body made from a porous ceramic such as alumina, mullite, zirconia, or cordierite or a porous sintered metal such as stainless steel is preferable. This is because a porous body made of a porous ceramic or a porous sintered metal has excellent mechanical strength.

The porous support may have any shape such as a flat film shape, a flat plate shape, a tube shape, or a honeycomb shape without any specific limitations.

The porous separation layer can be formed by, for example, synthesizing a zeolite having a desired structure, such as an MFI-type zeolite, on a porous support or on a porous support having zeolite seed crystals adhered thereto. Specifically, the porous separation layer can be formed on a porous support having zeolite seed crystals optionally adhered thereto by immersing the porous support in an aqueous sol containing a silica source and a structure directing agent, and synthesizing a zeolite by hydrothermal synthesis.

Note that in a situation in which a zeolite membrane obtained by forming a porous separation layer on a porous support is used in the presently disclosed membrane separation method, it is preferable that the zeolite membrane has been subjected to firing treatment to remove structure directing agent, and more preferable that the zeolite membrane has been subjected to boil washing and then firing treatment in an oxygen-containing atmosphere such as an air atmosphere.

<Step (A)>

In step (A), the zeolite membrane set forth above is exposed to an atmosphere having a dew point of -20°C or lower. This is because the separation efficiency in membrane separation of a hydrocarbon mixture in step (B) decreases if the zeolite membrane is not exposed to an atmosphere having a dew point of -20°C or lower.

It is preferable that an operation of raising the temperature of the atmosphere to which the zeolite membrane is exposed is performed in step (A). This is because raising the temperature of the atmosphere having a dew point of -20°C or lower to which the zeolite membrane is exposed can further increase the permeation flux F and separation factor $\alpha$, and further improve separation efficiency in membrane separation of a hydrocarbon mixture in step (B). In a situation in which the temperature of the atmosphere to which the zeolite membrane is exposed is raised in step (A), the temperature of the atmosphere may or may not also be lowered during implementation of step (A). However, from a viewpoint of inhibiting damage to the zeolite membrane caused by a rapid temperature drop during membrane separation of a hydrocarbon mixture in step (B), it is preferable that the temperature of the atmosphere to which the zeolite membrane is exposed is lowered during implementation of step (A), and it is particularly preferable that the temperature is lowered during implementation of step (A) in a situation in which the temperature of the atmosphere to which the zeolite membrane is exposed in step (A) is 350°C or higher.

[Atmosphere having dew point of -20°C or lower]

The atmosphere to which the zeolite membrane is exposed in step (A) may be any atmosphere having a dew point of -20°C or lower. Specifically, the atmosphere to which the zeolite membrane is exposed may be, but is not specifically limited to, an atmosphere composed of a gas that can pass through the zeolite membrane such as a dry air atmosphere, a carbon dioxide atmosphere, a nitrogen atmosphere, an argon atmosphere, or a helium atmosphere. Of such atmospheres, inert gas atmospheres such as a nitrogen atmosphere, an argon atmosphere, and a helium atmosphere are preferable as the atmosphere to which the zeolite membrane is exposed. This is because, in a situation in which the atmosphere to which the zeolite membrane is exposed is an inert gas atmosphere, reaction of atmosphere gas with the zeolite membrane to form an oxide or the like can be inhibited, and reduction of the permeation flux F and separation factor $\alpha$ can be prevented even if, for example, a zeolite membrane containing a reactive component is used, such as a zeolite membrane including solid acid sites.

Moreover, although no specific limitations are placed on the dew point of the atmosphere to which the zeolite membrane is exposed other than being -20°C or lower, the dew point is preferably -40°C or lower, and more preferably -50°C or lower from a viewpoint of further improving separation efficiency in membrane separation of a hydrocarbon mixture. Note that the dew point of the atmosphere to which the zeolite membrane is exposed is normally -80°C or higher.

Moreover, although no specific limitations are placed on the temperature of the atmosphere to which the zeolite membrane is exposed in step (A) other than being a higher temperature than the dew point of the atmosphere, the temperature of the atmosphere is preferably within a range of 10°C to 580°C, and more preferably within a range of 20°C to 580°C. Moreover, the maximum temperature of the atmosphere to which the zeolite membrane is exposed in step (A) is preferably 100°C or higher, and more preferably 130°C or higher, and is preferably 580°C or lower, and more preferably 500°C or lower. If the maximum temperature of the atmosphere to which the zeolite membrane is exposed is too low, it may not be possible to sufficiently improve separation efficiency in membrane separation of a hydrocarbon mixture. Conversely, if the maximum temperature of the atmosphere to which the zeolite membrane is exposed is too high, this may cause loss of the zeolite membrane and reduce the separation factor $\alpha$ in membrane separation of a hydrocarbon mixture. In a case in which a temperature raising operation is not performed, the maximum temperature of the atmosphere in step (A) is normally equivalent to the temperature of the atmosphere at the start of step (A).

The pressure (gauge pressure) of the atmosphere to which the zeolite membrane is exposed is, for example, preferably 1 MPa or lower. This is because the zeolite membrane may be damaged if the pressure is too high.

[Exposure conditions]

Although no specific limitations are placed on the time for which the zeolite membrane is exposed to the atmosphere having a dew point of -20°C or lower in step (A), the time is preferably 5 hours or more, more preferably 10 hours or more, and even more preferably 15 hours or more, and is normally 500 hours or less. Separation efficiency can be further improved when the time for which the zeolite membrane is exposed to the atmosphere having a dew point of -20°C or lower is at least any of the lower limits set forth above.

Moreover, although no specific limitations are placed on the time for which the zeolite membrane is exposed to the atmosphere at the aforementioned maximum temperature in step (A), the time is preferably 5 hours or more, more preferably 10 hours or more, and even more preferably 15 hours or more, and is preferably 50 hours or less, and more preferably 30 hours or less. By setting the time for which the atmosphere to which the zeolite membrane is exposed is held at the maximum temperature as at least any of the lower limits set forth above, the permeation flux F and separation factor $\alpha$ can be sufficiently increased, and separation efficiency can be further improved in membrane separation of a hydrocarbon mixture. Moreover, by setting the time for which the atmosphere to which the zeolite membrane is exposed is held at the maximum temperature as not more than any of the upper limits set forth above, loss of the zeolite membrane caused by heat and reduction of the separation factor $\alpha$ can be inhibited, and separation efficiency can be sufficiently improved in membrane separation of a hydrocarbon mixture.

The method by which the zeolite membrane is exposed to an atmosphere having a dew point of -20°C or lower may be any method so long as an atmosphere having a dew point of -20°C or lower can be provided as an atmosphere surrounding the zeolite membrane. Specifically, in step (A), the zeolite membrane may be exposed to an atmosphere having a dew point of -20°C or lower by, for example, continuously or intermittently feeding a gas having a dew point of -20°C or lower into a space in which the zeolite membrane is housed, or the zeolite membrane may be exposed to an atmosphere having a dew point of -20°C or lower by, for example, purging a space in which the zeolite membrane is housed with a gas having a dew point of -20°C or lower, and subsequently maintaining the space in an air-tight state.

The location where the zeolite membrane is exposed to the atmosphere having a dew point of -20°C or lower may, for example, be a location where membrane separation of a hydrocarbon mixture is performed in step (B), such as inside a membrane separation module that includes a housing with the zeolite membrane housed therein, or may, for example, be a storage vessel for storing the zeolite membrane outside of a membrane separation module. Of these examples, it is preferable that the zeolite membrane is exposed to the atmosphere having a dew point of -20°C or lower at a location where membrane separation of a hydrocarbon mixture is performed in step (B), such as inside a housing of a membrane separation module, from a viewpoint of quickly implementing step (B) after step (A) ends.

[Temperature raising operation]

In a situation in which an operation is performed of raising the temperature of the atmosphere to which the zeolite membrane is exposed in step (A), raising of the temperature may be started from the beginning of step (A), or may be started partway through step (A).

The temperature of the atmosphere is preferably raised to 100°C or higher, and more preferably to 130°C or higher, and is preferably raised to 580°C or lower, and more preferably to 500°C or lower. By setting the temperature of the atmosphere after being raised (i.e., the raised temperature) as at least any of the lower limits set forth above, the permeation flux F and separation factor $\alpha$ can be sufficiently increased, and separation efficiency can be sufficiently improved in membrane separation of a hydrocarbon mixture. Moreover, by setting the temperature of the atmosphere after being raised (raised temperature) as not higher than any of the upper limits set forth above, loss of the zeolite membrane caused by heat and reduction of the separation factor $\alpha$ can be inhibited, and separation efficiency can be sufficiently improved in membrane separation of a hydrocarbon mixture.

Moreover, although no specific limitations are placed on the time for which the temperature of the atmosphere is maintained at the raised temperature after being raised, this time is preferably 5 hours or more, more preferably 10 hours or more, and even more preferably 15 hours or more, and is preferably 50 hours or less, and more preferably 30 hours or less. By setting the time for which the atmosphere is maintained at the raised temperature as at least any of the lower limits set forth above, the permeation flux F and separation factor $\alpha$ can be sufficiently increased, and separation efficiency can be sufficiently improved in membrane separation of a hydrocarbon mixture. Moreover, by setting the time for which the atmosphere is maintained at the raised temperature as not more than any of the upper limits set forth above, loss of the zeolite membrane caused by heat and reduction of the separation factor $\alpha$ can be inhibited, and separation efficiency can be sufficiently improved in membrane separation of a hydrocarbon mixture.

[Temperature lowering operation]

In step (A), the temperature of the atmosphere that has been raised may, for example, be lowered to 30°C or lower, and preferably 25°C or lower. By lowering the temperature of the atmosphere to which the zeolite membrane is exposed to not higher than any of the upper limits set forth above, it is possible to inhibit damage to the zeolite membrane caused by a rapid temperature drop in membrane separation of a hydrocarbon mixture in step (B).

In a situation in which the temperature of the atmosphere is lowered, the timing at which step (A) ends may be simultaneous to the end of temperature lowering or may be after any amount of time has passed from the end of temperature lowering.

<Step (B)>

In step (B), membrane separation of a hydrocarbon mixture is performed using the zeolite membrane that has been exposed to an atmosphere having a dew point of -20°C or lower in step (A).

Step (B) is normally implemented following step (A) (i.e., straight after step (A)). In other words, in the presently disclosed membrane separation method, it is preferable that step (B) is implemented without the zeolite membrane being exposed to an atmosphere having a dew point of higher than -20°C after step (A). However, the presently disclosed membrane separation method may include any other step between step (A) and step (B) so long as the desired effects are obtained. Specifically, in a situation in which step (A) is implemented outside of a membrane separation module, for example, a step of assembling the zeolite membrane into a housing under an atmosphere having a dew point of higher than -20°C, such as an air atmosphere, may be included between step (A) and step (B) so long as the desired effects are obtained.

[Membrane separation]

In step (B), one or more hydrocarbon compounds contained in the hydrocarbon mixture are separated. Although no specific limitations are made, in one specific example, a linear hydrocarbon, for example, may be efficiently separated and removed from a hydrocarbon mixture including the linear hydrocarbon and a branched hydrocarbon and/or cyclic hydrocarbon of equivalent carbon number in step (B) such as to increase the percentage content of the branched hydrocarbon and/or cyclic hydrocarbon in the hydrocarbon mixture. More specifically, one or more components (for example, a linear hydrocarbon) may be separated and removed from the hydrocarbon mixture by passing the hydrocarbon mixture through the zeolite membrane in step (B).

Membrane separation using the zeolite membrane may be performed under any conditions and is preferably performed under heated conditions. Specifically, membrane separation is preferably performed under conditions of at least 20°C and not higher than 300°C, more preferably at least 25°C and not higher than 250°C, and even more preferably at least 50°C and not higher than 200°C. Although no specific limitations are placed on the pressure conditions under which membrane separation is performed, the pressure difference between the retentate side and the permeate side (pressure of retentate side - pressure of permeate side) is preferably at least 10 kPa and not more than 600 kPa, and more preferably at least 50 kPa and not more than 300 kPa.

The hydrocarbon mixture can be membrane separated with a high permeation flux F and a high separation factor $\alpha$ in step (B) because a zeolite membrane that has been exposed to an atmosphere having a dew point of -20°C or lower is used. Consequently, the hydrocarbon mixture can be membrane separated with high separation efficiency.

(Membrane separation device)

The presently disclosed membrane separation device includes: a membrane separation module including a housing and a zeolite membrane that is housed in the housing and is configured to perform membrane separation of a hydrocarbon mixture; a feedstock supply mechanism configured to supply the hydrocarbon mixture into the membrane separation module; and a gas supply mechanism configured to supply a gas having a dew point of -20°C or lower into a space in which the zeolite membrane is housed in the membrane separation module. By bringing the gas having a dew point of -20°C or lower into contact with the zeolite membrane using the gas supply mechanism, and subsequently supplying the hydrocarbon mixture into the membrane separation module using the feedstock supply mechanism in the presently disclosed membrane separation device, the hydrocarbon mixture can be membrane separated with high separation efficiency by the presently disclosed membrane separation method set forth above.

The presently disclosed membrane separation device preferably further includes a heating device configured to heat the gas having a dew point of -20°C or lower from a viewpoint of further improving separation efficiency of a hydrocarbon mixture.

<Membrane separation module>

The membrane separation module may, as illustrated for one example of a membrane separation device 100 according to the present disclosure in FIG. 1, be a membrane separation module 30 that includes a housing 31 and a zeolite membrane 32 that is housed in the housing 31 and defines a retentate-side region 33 and a permeate-side region 34 inside the housing.

The housing 31 of the membrane separation module 30 may be a known housing that can secure the zeolite membrane 32 in an air-tight manner.

Moreover, the zeolite membrane 32 may be the same zeolite membrane as in the presently disclosed membrane separation method set forth above.

A retentate outflow mechanism 60 for outflow of retentate is provided at a downstream side of the retentate-side region 33 of the membrane separation module 30. The retentate outflow mechanism 60 includes a retentate line 61, a back pressure valve 62, and a retentate line valve 63. The retentate line 61 may be connected to a retentate collection device (not illustrated), or may be connected to a storage tank 10 for the hydrocarbon mixture such as to form a circulation flow path.

A gas outflow line 71 for outflow of gas supplied from a gas supply mechanism branches and extends from the retentate line 61. A gas outflow line valve 72 is provided on the gas outflow line 71. The gas outflow line 71 and the gas outflow line valve 72 form a gas outflow mechanism 70 for outflow of gas that is supplied from a gas supply mechanism 40 and brought into contact with the zeolite membrane.

A permeate outflow mechanism 50 including a permeate line for outflow of permeate is provided at a downstream side of the permeate-side region 34 of the membrane separation module 30. A permeate line valve (not illustrated) is provided on the permeate line. The permeate line is connected to a permeate collection device (not illustrated) such as a cold trap.

<Feedstock supply mechanism>

Although no specific limitations are made, the feedstock supply mechanism may, for example, be a feedstock supply mechanism 20 that is configured to supply a hydrocarbon mixture to the membrane separation module 30 from the storage tank 10 for the hydrocarbon mixture as illustrated in FIG. 1. More specifically, the feedstock supply mechanism 20 may be a mechanism including a feedstock line 21 that connects the storage tank 10 for the hydrocarbon mixture to the retentate-side region 33 of the membrane separation module 30, a feeding device 22 provided on the feedstock line 21 that is configured to feed hydrocarbon mixture in the storage tank 10 to the retentate-side region 33, a heating device 23 provided on the feedstock line 21 that is configured to heat the hydrocarbon mixture, and a feedstock line valve 24.

No specific limitations are placed on the hydrocarbon mixture that is stored in the storage tank 10 and the same hydrocarbon mixture as in the presently disclosed membrane separation method, for example, may be stored.

The feeding device 22 may, for example, be a pump or the like.

Moreover, the heating device 23 may, for example, be a heat exchanger, a heater, or the like.

Through the feedstock supply mechanism 20, by operating the feeding device 22 and the heating device 23 with the feedstock line valve 24 in an open state, hydrocarbon mixture that is fed through the feeding device 22 can be heated and vaporized by the heating device 23, and can be supplied to the retentate-side region 33 of the membrane separation module 30. Moreover, through the feedstock supply mechanism 20, the supply of the hydrocarbon mixture to the retentate-side region 33 of the membrane separation module 30 can be stopped by closing the feedstock line valve 24.

<Gas supply mechanism>

Although no specific limitations are made, the gas supply mechanism may, for example, be a gas supply mechanism 40 that is configured to supply a gas (nitrogen gas in the illustrated example) having a dew point of -20°C or lower to the membrane separation module 30 from a gas supply source such as illustrated in FIG. 1. More specifically, the gas supply mechanism 40 may be a mechanism including a gas line 41 that is linked to the feedstock line 21 between the feedstock line valve 24 and the membrane separation module 30 and that connects a supply source (not illustrated) of a gas having a dew point of -20°C or lower and the retentate-side region 33 of the membrane separation module 30, a gas line valve 42, and a heating device 43 provided on the gas line 41 that is configured to heat the gas having a dew point of -20°C or lower.

Examples of the gas having a dew point of -20°C or lower include, but are not specifically limited to, gases that can pass through the zeolite membrane such as dry air, carbon dioxide gas, nitrogen gas, argon gas, and helium gas. Of these gases, inert gases such as nitrogen gas, argon gas, and helium gas are preferable. This is because, in a situation in which the gas that is brought into contact with the zeolite membrane is an inert gas, reaction of the gas with the zeolite membrane to form an oxide or the like can be inhibited, and reduction of the permeation flux F and separation factor $\alpha$ can be prevented even if, for example, a zeolite membrane containing a reactive component is used, such as a zeolite membrane including solid acid sites. The gas may be supplied from the supply source (not illustrated) using a compressor or the like.

Moreover, the heating device 43 may, for example, be a heat exchanger, a heater, or the like.

Through the gas supply mechanism 40, the gas can be heated by the heating device 43 and can be supplied to the retentate-side region 33 of the membrane separation module 30 by operating the heating device 43 with the gas line valve 42 in an open state. Moreover, through the gas supply mechanism 40, the supply of the gas to the retentate-side region 33 of the membrane separation module 30 can be stopped by closing the gas line valve 42.

Through the membrane separation device 100 set forth above, a gas (nitrogen gas in the illustrated example) having a dew point of -20°C or lower can be supplied to the membrane separation module 30 of the gas supply mechanism 40 with the feedstock line valve 24, the retentate line valve 63, and the permeate line valve (not illustrated) in a closed state and with the gas line valve 42 and the gas outflow line valve 72 in an open state. Specifically, gas having a dew point of -20°C or lower that has been heated by the heating device 43 can be fed into the housing 31 of the membrane separation module 30 and the heated gas having a dew point of -20°C or lower can be brought into contact with the zeolite membrane 32. Gas that has been brought into contact with the zeolite membrane 32 may subsequently be discharged to any processing device via the gas outflow line 71.

Moreover, through the membrane separation device 100 set forth above, after the gas having a dew point of -20°C or lower has been brought into contact with the zeolite membrane 32 as described above, the feedstock line valve 24, the retentate line valve 63, and the permeate line valve (not illustrated) may be opened, and the gas line valve 42 and the gas outflow line valve 72 may be closed so that the hydrocarbon mixture can be fed from the feedstock supply mechanism 20 to the membrane separation module 30, and membrane separation of the hydrocarbon mixture can be performed. Specifically, vaporized hydrocarbon mixture can be fed to the retentate-side region 33 of the membrane separation module 30 through the feeding device 22 and the heating device 23, permeate that passes through the zeolite membrane 32 can be collected through the permeate outflow mechanism 50, and retentate that does not pass through the zeolite membrane 32 can be collected or circulated through the retentate outflow mechanism 60.

Accordingly, through the membrane separation device 100, a hydrocarbon mixture can be membrane separated with high separation efficiency by the presently disclosed membrane separation method set forth above.

It should be noted that although the presently disclosed membrane separation device is described above using one example, the presently disclosed membrane separation device is not limited to the configuration illustrated in FIG. 1. Specifically, the heating device 43 configured to heat the gas having a dew point of -20°C or lower may alternatively be provided at the periphery of the housing 31 of the membrane separation module 30 or inside the housing 31 instead of in the gas supply mechanism 40. However, it is preferable that the heating device 43 is provided in the gas supply mechanism 40 from a viewpoint of bringing preheated gas into contact with the zeolite membrane 32 so as to uniformly heat the zeolite membrane, and further improving separation efficiency in membrane separation of a hydrocarbon mixture. Moreover, the gas line 41 may be directly connected to the retentate-side region 33 without being connected to the feedstock line 21, may be connected to the permeate-side region 34, or may be connected to both the retentate-side region 33 and the permeate-side region 34. Furthermore, the gas outflow mechanism 70 including the gas outflow line 71 and the gas outflow line valve 72 may be provided at the permeate outflow mechanism 50 side without being provided at the retentate outflow mechanism 60 side, or may be provided at both the permeate outflow mechanism 50 side and the retentate outflow mechanism 60 side.

EXAMPLES

The following provides a more specific description of the present disclosure based on examples. However, the present disclosure is not limited to the following examples. In the following description, "%" and the like used to express quantities are by mass, unless otherwise specified.

The separation efficiency of a hydrocarbon mixture in the examples and comparative examples was measured and evaluated by the following method.

<Separation efficiency>

{Examples 1 to 3 and Comparative Example 1}

The permeation flux F was calculated from the results of a membrane separation test using the following equation (I). Moreover, the separation factor $\alpha$ was calculated using the following equation (II). Furthermore, F $\times$ $\alpha$ was calculated, and separation efficiency was evaluated. Larger values for F, $\alpha$, and F $\times$ $\alpha$ indicate better separation efficiency.

$$F = W/(A \times t) \qquad\qquad (I)$$

$$\alpha = (Y_n/Y_{iso})/(X_n/X_{iso}) \qquad\qquad (II)$$

In equation (I), W is the mass (kg) of components that pass through the zeolite membrane, A is the effective area (m$^2$) of the zeolite membrane, and t is the processing time (h). Moreover, in equation (II), $X_n$ is the percentage content (mol%) of n-pentane in a feedstock, $X_{iso}$ is the percentage content (mol%) of isopentane in the feedstock, $Y_n$ is the percentage content (mol%) of n-pentane in a permeate side sample, and $Y_{iso}$ is the percentage content (mol%) of isopentane in the permeate side sample.

{Examples 4 to 6 and Comparative Example 2}

The permeation flux F was calculated from the results of a membrane separation test using the following equation (I). Moreover, the separation factor $\alpha$ was calculated using the following equation (II'). Furthermore, F $\times$ $\alpha$ was calculated, and separation efficiency was evaluated. Larger values for F, $\alpha$, and F $\times$ $\alpha$ indicate better separation efficiency.

$$F = W/(A \times t) \qquad\qquad (I)$$

$$\alpha = (Y_n/Y_{iso+cyclo})/(X_n/X_{iso+cyclo}) \qquad\qquad (II')$$

In equation (I), W is the mass (kg) of components that pass through the zeolite membrane, A is the effective area (m$^2$) of the zeolite membrane, and t is the processing time (h). Moreover, in equation (II'), $X_n$ is the percentage content (mol%) of linear hydrocarbon having a carbon number of 5 in a feedstock, $X_{iso+cyclo}$ is the total percentage content (mol%) of branched hydrocarbon having a carbon number of 5 and cyclic hydrocarbon having a carbon number of 5 in the feedstock, $Y_n$ is the percentage content (mol%) of linear hydrocarbon having a carbon number of 5 in a permeate side sample, and $Y_{iso+cyclo}$ is the total percentage content (mol%) of branched hydrocarbon having a carbon number of 5 and cyclic hydrocarbon having a carbon number of 5 in the permeate side sample

(Example 1)

<Membrane separation test>

A membrane separation test was carried out using a membrane separation device 100 such as illustrated in FIG. 1 and using an MFI-type zeolite membrane that was obtained by forming a porous separation layer composed of an MFI-type zeolite on the outer surface of a circular tube-shaped porous support made of mullite, and then performing firing for 20 hours at a temperature of 500°C in an air atmosphere having a dew point of 2°C to remove structure directing agent. The permeate line of the membrane separation device 100 was connected to a cold trap for sampling, and the retentate line 61 of the membrane separation device 100 was connected to the storage tank 10 via a heat exchanger as a cooling device.

[Membrane separation]

The membrane separation test using the membrane separation device 100 illustrated in FIG. 1 was implemented as follows.
Specifically, the storage tank 10 was first filled with a C5 hydrocarbon mixture composed of a mixed liquid of n-pentane and isopentane (mixed liquid comprising 50 mol% of n-pentane and 50 mol% of isopentane), and a degassing operation was performed three times.

Next, nitrogen gas (dew point: -50°C) was fed to the membrane separation module 30 from the gas supply mechanism 40 with the feedstock line valve 24, the retentate line valve 63, and the permeate line valve (not illustrated) in a closed state and the gas line valve 42 and the gas outflow line valve 72 in an open state, and the nitrogen gas was brought into contact with the zeolite membrane 32. Specifically, nitrogen gas that had been heated by the heating device 43 was fed into the housing 31 of the membrane separation module 30, the temperature inside the housing 31 was raised to 500°C (maximum temperature), and then nitrogen gas was brought into contact with the zeolite membrane 32 for 15 hours at the maximum temperature of 500°C. The temperature inside the housing 31 was subsequently lowered to 20°C, and nitrogen gas was brought into contact with the zeolite membrane 32 for 19 hours at a temperature of 20°C.

Thereafter, the feedstock line valve 24 and the retentate line valve 63 were opened, and the gas line valve 42 and the gas outflow line valve 72 were closed. Next, a feedstock circulation process was started in which the hydrocarbon mixture was heated to 70°C by the heating device 23, was supplied to the membrane separation module 30 in the gas phase, and was subsequently condensed using the cooling device and returned to the storage tank 10. Operation was continued after starting the feedstock circulation process until the temperature in the system reached a steady state. Once the temperature in the system reached a steady state, the retentate side was pressurized to 150 kPa (gauge pressure) by the back pressure valve 62 and the permeate side (cold trap) was depressurized to -100 kPa (gauge pressure). Once stabilization of the temperature and pressure in the system was confirmed, the permeate line valve (not illustrated) was opened, and the membrane separation test was started. In other words, the membrane separation test was carried out under conditions of a temperature of 70°C and a pressure difference between the retentate side and the permeate side of 250 kPa.

Extraction of a permeate side sample was started from the point at which 5 minutes had passed from the start of the membrane separation test. The permeate side sample (condensate) was weighed and the molar ratio of n-pentane and isopentane was measured using a gas chromatograph. These measurement results were used to evaluate the separation efficiency. The results are shown in Table 1.

(Example 2)

A membrane separation test was carried out in the same way as in Example 1 with the exception that the maximum temperature was changed to 150°C. The results are shown in Table 1.

(Example 3)

A membrane separation test was carried out in the same way as in Example 1 with the exception that the maximum temperature was changed to 200°C. The results are shown in Table 1.

(Comparative Example 1)

A membrane separation test was carried out in the same way as in Example 1 with the exception that membrane separation of the hydrocarbon mixture was performed without feeding of nitrogen gas in the membrane separation test. The results are shown in Table 1.

Table 1

| | | Example 1 | Example 2 | Example 3 | Comparative Example 1 |
|---|---|---|---|---|---|
| Zeolite membrane | Type | MFI-type zeolite | MFI-type zeolite | MFI-type zeolite | MFI-type zeolite |
| Step (A) | Atmosphere/ Dew point | $N_2$/-50°C | $N_2$/-50°C | $N_2$/-50°C | Not implemented |
| | Maximum temperature (°C) | 500 | 150 | 200 | |
| | Holding time (h) | 15 | 15 | 15 | |

(continued)

|  |  | Example 1 | Example 2 | Example 3 | Comparative Example 1 |
|---|---|---|---|---|---|
| Zeolite membrane | Type | MFI-type zeolite | MFI-type zeolite | MFI-type zeolite | MFI-type zeolite |
| Evaluation | Separation factor $\alpha$ | 31 | 31 | 29 | 22 |
|  | Permeation flux F (kg/m$^2$·h) | 5.47 | 5.49 | 4.46 | 1.95 |
|  | $\alpha \times F$ | 170 | 171 | 131 | 43 |

It can be seen from Table 1 that separation efficiency is improved in Examples 1 to 3 compared to Comparative Example 1.

(Example 4)

<Membrane separation test>

A membrane separation test was carried out using a membrane separation device 100 such as illustrated in FIG. 1 and using an MFI-type zeolite membrane that was obtained by forming a porous separation layer composed of an MFI-type zeolite on the outer surface of a circular tube-shaped porous support made of mullite, and then performing firing for 20 hours at a temperature of 500°C in an air atmosphere having a dew point of 2°C to remove structure directing agent. The permeate line of the membrane separation device 100 was connected to a cold trap for sampling, and the retentate line 61 of the membrane separation device 100 was connected to the storage tank 10 via a heat exchanger as a cooling device.

[Membrane separation]

The membrane separation test using the membrane separation device 100 illustrated in FIG. 1 was implemented as follows.
Specifically, the storage tank 10 was first filled with a C5 hydrocarbon mixture composed of a fraction remaining after some isoprene had been collected from a C5 fraction obtained as a by-product in production of ethylene by thermal cracking of naphtha, and a degassing operation was performed three times. (Note that the remaining fraction was a mixture containing linear hydrocarbon having a carbon number of 5, branched hydrocarbon having a carbon number of 5, and cyclic hydrocarbon having a carbon number of 5 as main components.)
Next, nitrogen gas (dew point: -50°C) was fed to the membrane separation module 30 from the gas supply mechanism 40 with the feedstock line valve 24, the retentate line valve 63, and the permeate line valve (not illustrated) in a closed state and the gas line valve 42 and the gas outflow line valve 72 in an open state, and the nitrogen gas was brought into contact with the zeolite membrane 32. Specifically, nitrogen gas that had been heated by the heating device 43 was fed into the housing 31 of the membrane separation module 30, the temperature inside the housing 31 was raised to 500°C (maximum temperature), and then nitrogen gas was brought into contact with the zeolite membrane 32 for 15 hours at the maximum temperature of 500°C. The temperature inside the housing 31 was subsequently lowered to 20°C, and nitrogen gas was brought into contact with the zeolite membrane 32 for 19 hours at a temperature of 20°C.
Thereafter, the feedstock line valve 24 and the retentate line valve 63 were opened, and the gas line valve 42 and the gas outflow line valve 72 were closed. Next, a feedstock circulation process was started in which the hydrocarbon mixture was heated to 70°C by the heating device 23, was supplied to the membrane separation module 30 in the gas phase, and was subsequently condensed using the cooling device and returned to the storage tank 10. Operation was continued after starting the feedstock circulation process until the temperature in the system reached a steady state. Once the temperature in the system reached a steady state, the retentate side was pressurized to 150 kPa (gauge pressure) by the back pressure valve 62 and the permeate side (cold trap) was depressurized to -100 kPa (gauge pressure). Once stabilization of the temperature and pressure in the system was confirmed, the permeate line valve (not illustrated) was opened, and the membrane separation test was started. In other words, the membrane separation test was carried out under conditions of a temperature of 70°C and a pressure difference between the retentate side and the permeate side of 250 kPa.
Extraction of a permeate side sample was started from the point at which 55 minutes had passed from the start of the membrane separation test. The permeate side sample (condensate) was weighed and the molar ratio of linear hydrocarbon having a carbon number of 5, branched hydrocarbon having a carbon number of 5, and cyclic hydrocarbon having a carbon number of 5 was measured using a gas chromatograph. These measurement results were used to evaluate the separation efficiency. The results are shown in Table 2.

(Example 5)

A membrane separation test was carried out in the same way as in Example 4 with the exception that the maximum temperature was changed to 150°C. The results are shown in Table 2.

(Example 6)

A membrane separation test was carried out in the same way as in Example 4 with the exception that the maximum temperature was changed to 200°C. The results are shown in Table 2.

(Comparative Example 2)

A membrane separation test was carried out in the same way as in Example 4 with the exception that membrane separation of the hydrocarbon mixture was performed without feeding of nitrogen gas in the membrane separation test. The results are shown in Table 2.

Table 2

| | | Example 4 | Example 5 | Example 6 | Comparative Example 2 |
|---|---|---|---|---|---|
| Zeolite membrane | Type | MFI-type zeolite | MFI-type zeolite | MFI-type zeolite | MFI-type zeolite |
| Step (A) | Atmosphere/ Dew point | $N_2$/-50°C | $N_2$/-50°C | $N_2$/-50°C | Not implemented |
| | Maximum temperature (°C) | 500 | 150 | 200 | |
| | Holding time (h) | 15 | 15 | 15 | |
| Evaluation | Separation factor $\alpha$ | 32 | 38 | 40 | 26 |
| | Permeation flux F ($kg/m^2 \cdot h$) | 4.19 | 3.50 | 3.22 | 2.03 |
| | $\alpha \times F$ | 134 | 133 | 129 | 53 |

It can be seen from Table 2 that even when a fraction remaining after some isoprene is collected from a C5 fraction is used, separation efficiency is improved in Examples 4 to 6 compared to Comparative Example 2.

INDUSTRIAL APPLICABILITY

According to the present disclosure, it is possible to provide a membrane separation method and a membrane separation device that enable membrane separation of a hydrocarbon mixture with high separation efficiency.

REFERENCE SIGNS LIST

10      storage tank
20      feedstock supply mechanism
21      feedstock line
22      feeding device
23      heating device
24      feedstock line valve
30      membrane separation module

| 31 | housing |
| 32 | zeolite membrane |
| 33 | retentate-side region |
| 34 | permeate-side region |
| 40 | gas supply mechanism |
| 41 | gas line |
| 42 | gas line valve |
| 43 | heating device |
| 50 | permeate outflow mechanism |
| 60 | retentate outflow mechanism |
| 61 | retentate line |
| 62 | back pressure valve |
| 63 | retentate line valve |
| 70 | gas outflow mechanism |
| 71 | gas outflow line |
| 72 | gas outflow line valve |
| 100 | membrane separation device |

**Claims**

1. A membrane separation method comprising:

   a step (A) of exposing a zeolite membrane to an atmosphere having a dew point of -20°C or lower; and
   a step (B) of using the zeolite membrane to perform membrane separation of a hydrocarbon mixture after the step (A).

2. The membrane separation method according to claim 1, wherein temperature of the atmosphere is raised in the step (A).

3. The membrane separation method according to claim 1 or 2, wherein
   the atmosphere has a maximum temperature of at least 100°C and not higher than 580°C in the step (A).

4. The membrane separation method according to any one of claims 1 to 3, wherein
   the atmosphere is an inert gas atmosphere.

5. The membrane separation method according to any one of claims 1 to 4, wherein
   the zeolite membrane is exposed to the atmosphere for 5 hours or more in the step (A).

6. A membrane separation device comprising:

   a membrane separation module including a housing and a zeolite membrane that is housed in the housing and is configured to perform membrane separation of a hydrocarbon mixture;
   a feedstock supply mechanism configured to supply the hydrocarbon mixture into the membrane separation module; and
   a gas supply mechanism configured to supply a gas having a dew point of -20°C or lower into a space in which the zeolite membrane is housed in the membrane separation module.

7. The membrane separation device according to claim 6, further comprising a heating device configured to heat the gas.

8. The membrane separation device according to claim 7, wherein the gas supply mechanism includes the heating device.

9. The membrane separation device according to any one of claims 6 to 8, wherein
   the gas is an inert gas.

# FIG. 1

## INTERNATIONAL SEARCH REPORT

| | International application No. |
| --- | --- |
| | PCT/JP2017/005179 |

### A. CLASSIFICATION OF SUBJECT MATTER

$B01D71/02$(2006.01)i, $B01D53/22$(2006.01)i, $C07C7/144$(2006.01)i, $C07C9/18$(2006.01)i

According to International Patent Classification (IPC) or to both national classification and IPC

### B. FIELDS SEARCHED

Minimum documentation searched (classification system followed by classification symbols)
$B01D71/02$, $B01D53/22$, $C07C7/144$, $C07C9/18$

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)
JSTPlus/JMEDPlus/JST7580(JDreamIII), CAplus/REGISTRY(STN)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | CORONAS J. et al., Separations of $C_4$ and $C_6$ Isomers in ZSM-5 Tubular Membranes, Ind. Eng. Chem. Res., 1998.01.05, V37, P166-176, P167 "Permeation Measurements", P170-171 "Change in n-Butane/Isobutane Separations with time", Figure 9, P175 "Effect of Water" | 1-9<br>1-9 |
| X<br>Y | WO 2011/109607 A1 (SHELL INTERNATIONALE RESEARCH MAATSCHAPPIJ B.V.), 09 September 2011 (09.09.2011), claims 1, 7, 9, 12 to 13, 15; page 3, line 23 to page 4, line 5; page 7, line 28 to page 8, line 8<br>& US 2013/0000484 A1 | 1-9<br>1-9 |

☒ Further documents are listed in the continuation of Box C.   ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 30 March 2017 (30.03.17) | 11 April 2017 (11.04.17) |

| Name and mailing address of the ISA/<br>Japan Patent Office<br>3-4-3,Kasumigaseki,Chiyoda-ku,<br>Tokyo 100-8915,Japan | Authorized officer |
| --- | --- |
| | Telephone No. |

Form PCT/ISA/210 (second sheet) (January 2015)

**INTERNATIONAL SEARCH REPORT**

| International application No. |
|---|
| PCT/JP2017/005179 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2015-160186 A  (Nippon Zeon Co., Ltd.), 07 September 2015 (07.09.2015), claims 1 to 2; paragraphs [0042] to [0046], [0059]; fig. 1; examples (Family: none) | 1-9 |
| A | JP 2002-282640 A  (Kyocera Corp.), 02 October 2002 (02.10.2002), claims 1 to 2; paragraphs [0004], [0018], [0022] to [0023]; examples (Family: none) | 1-9 |
| P,X | JP 2016-159211 A  (Mitsubishi Chemical Corp.), 05 September 2016 (05.09.2016), claims 1 to 5; paragraphs [0003], [0036], [0048] to [0053], [0060]; fig. 1 to 5 (Family: none) | 1-9 |
| P,Y | WO 2016/121377 A1  (Nippon Zeon Co., Ltd.), 04 August 2016 (04.08.2016), claims 1 to 3; examples; fig. 1 (Family: none) | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014069630 A1
- JP 2015160186 A